# EUROPEAN PATENT APPLICATION

(11) **EP 1 913 897 A1**
(43) Date of publication of application: **23.04.2008**
(21) Application number: 05792175.1
(22) Date of filing: 22.09.2005
(51) Int. Cl.: A61F 2/06

(54) **A SURGICAL STENT PEOSTHESIS AND A TRANSPORTER FOR TRANSPORTING THE PEOSTHESES**

(30) Priority: 09.08.2005 CN 200510028616
(71) Applicant: Microport Medical (Shanghai) Co., Ltd., 201203 Shanghai (CN)
(72) Inventor: KE, Danian, Shanghai 201203 (CN); SUN, Lizhong, Shanghai 201203 (CN); ZHANG, Yi, Shanghai 201203 (CN); LUO, Qiyi, Shanghai 201203 (CN); MEI, Qifeng, Shanghai 201203 (CN); XIANG, Yonggang, Shanghai 201203 (CN)
(74) Representative: Weller, Wolfgang
(86) International application number: PCT/CN2005/001537
(87) International publication number: WO 2007/016821

(57) **Abstract**

The invention provides a kind of vascular stent graft used in surgical operation. It comprises a synthetic vascular graft and a self-expandable stent which is fixed inside the synthetic vascular graft, wherein the stent's outer wall keeps in contact with the inner wall of the synthetic vascular graft and the vascular graft's one end is longer than the corresponding end of the self-expandable stent. The invention provides also a delivery system used to deliver the surgical stent graft which can be in sheath form or in string controlled form.

The surgical stent graft according to the invention can be used in aortic aneurysm surgical operation. It can deal with the aortic section which can not be touched in the operation. The Surgical stent graft can be sewed with both synthetic vascular graft used for conventional surgery and vessels so that the surgical area is enlarged. The surgery can be fulfilled one time instead of two times like using traditional method.

## Description

### Field of the Invention

The invention relates to an endovascular implant grafts and implanting device used in vascular surgery. In particular, it relates to a stent graft used in surgical operation, and its delivery system.

### Background of the Invention

According to current medical practices, patients who contract an aortic aneurysm and require replacing the damaged section, including pars ascendens aortae, arcus aortae, and descending thoracic aorta, with a synthetic blood vessel graft requires an central breast open surgery. However, some areas of the aorta, such as descending thoracic aorta covered by the heart, can not be reached during the operation. Therefore, a second operation is needed to treat the descending thoracic aorta. To deal with this section, the traditional method is to insert a piece of vascular graft (elephant trunk) into the descending thoracic aorta through the cutting port. The function of this piece of vascular graft can make it easy to connect when secondary surgery cutting from other place performed. In this way, patients have to suffer from secondary surgery. Usually, the opening cutting port is situated under the fourth rib for secondary surgery. In the period between the two surgeries, there exist a lot of risks.

The purpose of the invention is to make a surgical stent graft and its delivery system which can help the surgery being fulfilled one time and thereby overcome the above defects.

### Summary of the Invention

According to the present invention, a surgical stent graft includes a self-expandable stent and synthetic vascular graft. The stent is fixed inside the vascular graft. The outside wall of the stent gets in touch with the inside wall of the vascular graft. One end of the vascular graft is longer than that of the stent. The stent can be sewed together with the vascular graft. The longer part of the vascular graft extending from the stent can make it easy to be sewed together with other vessel or vascular graft.

As further improvement of the invention, two ends of the synthetic vascular graft are longer than those of the stent.

As further improvement of the invention, there is a window in the stent-graft which divides the synthetic vascular graft and the stent respectively into two parts. The two parts of the self-expandable stent are connected by alloy wire. The curvature of the wire is the same as that of the stent vascular graft. The wire can be seen through the window.

As further improvement of the invention, there is a window in the vascular graft in the overlapping area of the vascular graft and the self-expandable stent. The self-expendable stent is visible through the window. The window can be of the shape of ellipse or other shapes.

Or in the middle section there is no vascular graft. That is to say that the window is arranged cylindrically along the circumference of synthetic vascular graft and divides the synthetic vessel into two separate parts. The self-expendable stent is visible through the window.

As further improvement of the invention, the synthetic vascular graft has 1-3 branch vessels so that every branch graft can be planted into each of branch vessels correspondingly.

The materials used for the synthetic vascular graft are chosen from polymers, such as PET, PTFE and etc. The stent is made of elastic metal materials which has superior bio-compatibility, such as Nitinol shape memory alloy, CoCr alloy, or 316L/317L stainless steel.

Another object of the invention is to provide a delivery system to delivery the surgical stent graft. This system comprises a handle with one end having a tube port, a shaft and a piece of string with one end fixed on the handle and the other end fixed on the shaft, wherein one end of the stent graft is inserted into the tube port and bound on the shaft by the string, and said shaft extends through the tube port. This system is characterized in that further comprising a core bar with a core bar hole, wherein said core bar is provided with a bullet-like tip at its head, and said handle is provided with a through hole in which the end of said core bar is fixed, and external guide wire can extend over the tip through the core bar hole. The bullet like tip can make it easy to thrust the surgical stent graft into the vessel, and protect the vessel from been damaged. When the true lumen of vessel at the cut port is pressed very small, the guide wire can be used to thrust into the vessel in advanced. The graft can go into the true lumen of the vessel over the wire. Thus make the operation is easier and reliable.

As further improvement of the invention, the delivery system comprises further a pistol-like body. Said handle is connected fixedly at the end with the muzzle, and the end of the shaft is connected with the trigger of the pistol. When the trigger is pulled repeatedly, the stent graft will release gradually, thereby the stent graft can be operated during the delivery and release procedure by one hand instead of two hands. The other spare hand can fix the cut port of the aortic vessel by surgery instruments so that to make the delivery and release operation easier and more precise and thus to improve the effectiveness.

It is also a good way to design sheath form delivery system. This system comprises a delivery sheath and a push shaft. This system comprises further a core bar with a core bar hole, wherein said core bar is provided with a bullet-like tip at its head, the other end of the core bar extends into the push shaft and connected fixedly with the push shaft, and the push shaft is provided inside with a through hole through which external guide wire can extend over the tip. The bullet like tip can make it easy to thrust the surgical stent graft into the vessel, and protect the vessel from been damaged. When the true lumen of vessel at the cut port is pressed very small, the guide wire can be used to thrust into the vessel in advanced. The graft can go into the vessel over the wire. Thus make the operation is easier and reliable.

As further improvement of the sheath form delivery system, the system comprises further a pistol-like body. Said delivery sheath is connected fixedly at the end with the muzzle, and the end of the push shaft is connected fixedly with the trigger of the pistol. The stent graft is pre-compressed between the delivery sheath and the core bar. In operation, insert the delivery sheath into the cut port of the aortic vessel to be treated, fix the handle of the push shaft by one hand and withdraw the delivery sheath rearwards by the other hand, thereby the stent graft can be released from the delivery sheath and expand automatically. And then scissor the cut port to be aligned and overlapped with the end of the surgical stent graft. The surgery is finished after sewing a piece of synthetic vessel replacing the aorta and this overlapping port.

### The present invention has the following advantage compared with the prior art:

According to the invention, unlike the conventional operation, it is unnessary to substitute the ascendens aorta and arcus aorta by synthetic vascular graft and then to sew the vascular graft together with the stent graft. Further, it is not necessary to sew. In this way a lot of time and the expenses can be saved, and the surgery is made easier. It is unnecessary to cut away the pathological synthetic vessel. The only thing which needs to do is to cut a port on the pathological vessel and thrust the stent graft into it. Moreover, the middle exposed stent graft can make its exposed part align with the three vessel branches of aorta and arcus, or thrust the three branches of the three-branch stent graft into the corresponding vessel branches of aorta and arcus. The proximal part of the graft corresponds to the ascendens aorta, the distal part of the graft corresponds to the dscendens aorta. So one stent graft can play both roles of the synthetic vessel and the surgical stent graft. Thus make the operation more effective and save more expenses. The surgical stent graft with window connected by two alloy wire has the advantage of decreasing the stimulation of the bare stent to the inside wall of the vessel.

The stent graft according to the invention has simple structure, easiness of use, reliable operational ability, retainableness of good geometry shape in blood vessel, good mechanical properties and excellent bio-compatibility, and adaptability of different structures for different pathological changes. It not only can fulfill two surgeries one time, but also save the surgical operation time for one surgery.

### Brief Description of Drawings

Fig.1. shows a surgical stent graft according to the present invention in a schematic view.
Fig.2. shows a surgical stent graft connected by metal wire in a schematic view, with its middle part exposed.
Fig.3. shows a surgical stent graft in a schematic view, with its middle part exposed.
Fig.4. shows a surgical stent graft in a schematic view, with its middle part partly exposed.
Fig.5. shows a surgical stent graft with three vessel branches in a schematic view.
Fig.6. shows a string controlled delivery system in a schematic view.
Fig.7. shows a string controlled delivery system with a pistol handle in a schematic view.
Fig.8. shows a delivery system with delivery sheath and pistol handle in a schematic view.
Fig.9. shows an embodiment of the stent graft according to the invention used in descending aorta in a schematic view.
Fig.10. shows an embodiment of the stent graft according to the invention used in abdominal aorta in a schematic view.
Fig.11. shows an embodiment of the stent graft according to the invention used in arcus aorta in a schematic view, with its middle part fullly exposed.
Fig.12. shows an embodiment of the stent graft according to the invention used in arcus aorta in a schematic view, with its middle part partly exposed.
Fig.13. shows an embodiment of the stent graft with three vessel branches according to the invention used in arcus aorta in a schematic view.

### Detail Description of Embodiments

The invention will be explained in details with reference to the drawings.

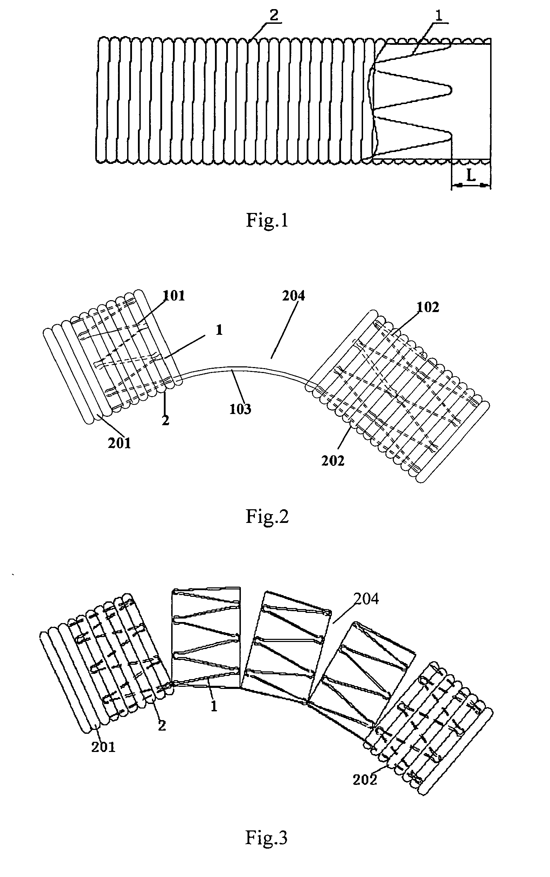

Fig.1 shows a surgical stent graft according to the invention, which comprises a synthetic vascular graft 2 and a self-expandable stent 1 which is arranged fixedly inside the vascular graft 2. The outer wall of the stent 1 keeps in contact with the inner wall of the vascular graft 2. The extra length at one end of the vascular graft 2 is L, which can be about 10mm, longer than that corresponding end of the stent 1. It is also possible to make both ends of the vascular graft longer than those corresponding ends of the stent 1.

Fig.2 shows a further improvement of the invention, in which there is a window 204 in the surgical stent graft, said window 204 divides the synthetic vascular graft 2 into two separate parts 201, 202 and divides the self-expandable stent 1 into two separate parts 101, 102. These two parts 101, 102 are connected by alloy wire 103. The curvature of the wire 103 is about the same as that of the vascular graft 2. The wire 103 is visible through the window 204.

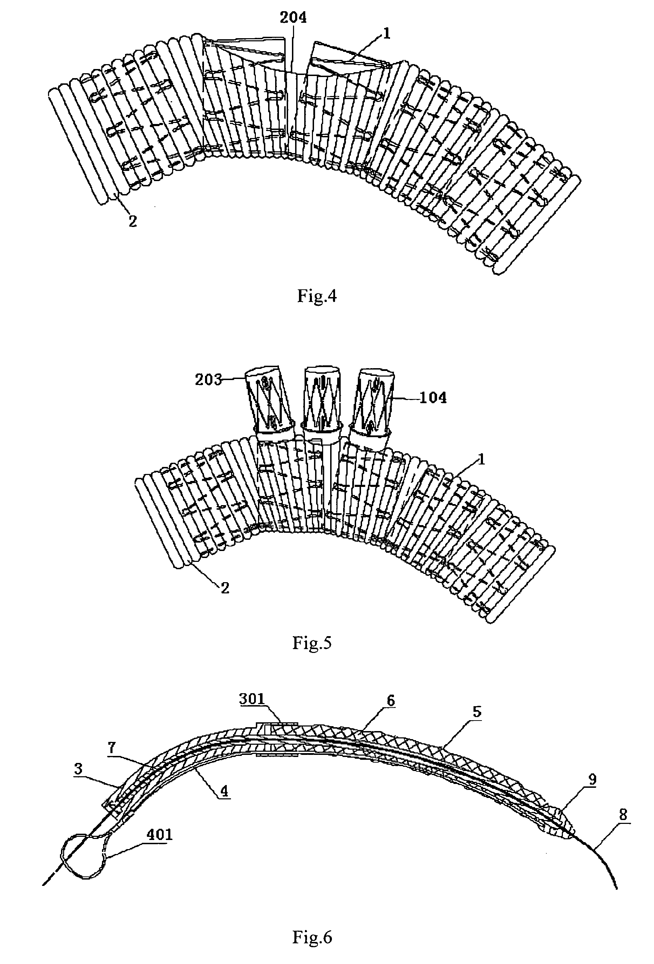

Fig.4 shows a further improvement of the invention, in which there is a window 204 in the overlapping area of the self-expandable stent 1 on the vascular graft 2. The self-expendable stent 1 is visible through the window 204. Said window 204 has a shape of ellipse.

As further Improvement of the invention shown in Fig. 3, the window is arranged around the vascular graft cylindrically and divides the vascular graft 2 into two separate parts 201, 202.The self-expandable stent 1 is exposed by the cylindrical window and visible through the window.

As further improvement of the invention shown in Fig. 5, the synthetic vascular graft 2 can have 1~3 branch vascular graft 203,104 so that a branch graft 203,104 can be planted correspondingly into each of the branch vessels.

The self-expandable stent has a Z-structure or woven structure and can bend towards not only one direction but also several directions in order to compromise the crooked vessels.

The synthetic vessel can be made by polymers, such as PET, PTFE and ect. The surface of the synthetic vessel can be smooth or corrugated.

Fig.6 shows a delivery system according to the invention to deliver the surgical stent graft. This delivery system includes a handle 3, a piece of string 5 and a shaft 4. One end of the handle is provided with a tube port 301. The string 5 is fixed at one end on the shaft 4 and at the other end on the handle 3. One end of the stent graft 6 is inserted into the tube port 301 and bound on the shaft 4 by the string 5, and said shaft 4 extends through the tube port 301 of the handle. This delivery system further comprises a core bar 7 with a core bar hole and a guide wire 8, wherein said core bar is provided with a bullet-like tip 9 at its head. Said handle 3 is provided with a through hole in which the end of said core bar 7 is fixed, and external guide wire 8 can extend over the tip 9 through the core bar hole. The shaft 4 is formed by a stainless metal wire and has a ring handle 401 at its end. In operation, insert the stent graft 6 into the aortic cut port to be treated; fix the handle 3 by one hand and pull the stainless metal shaft 4 by the other hand, and then the stent graft will release and expand automatically away from the tube port 301 of the handle.

As further improvement of the delivery system according to the invention, the delivery system comprises further a pistol-like body 10. The handle 3 is connected fixedly at its end with the muzzle 11 of the pistol-like body, and the end of the shaft 4 is connected fixedly with the trigger 12 of the pistol. When the trigger is pulled in use, the stent will be released and expand gradually. The whole procedure can be operated by one hand instead of two hands.

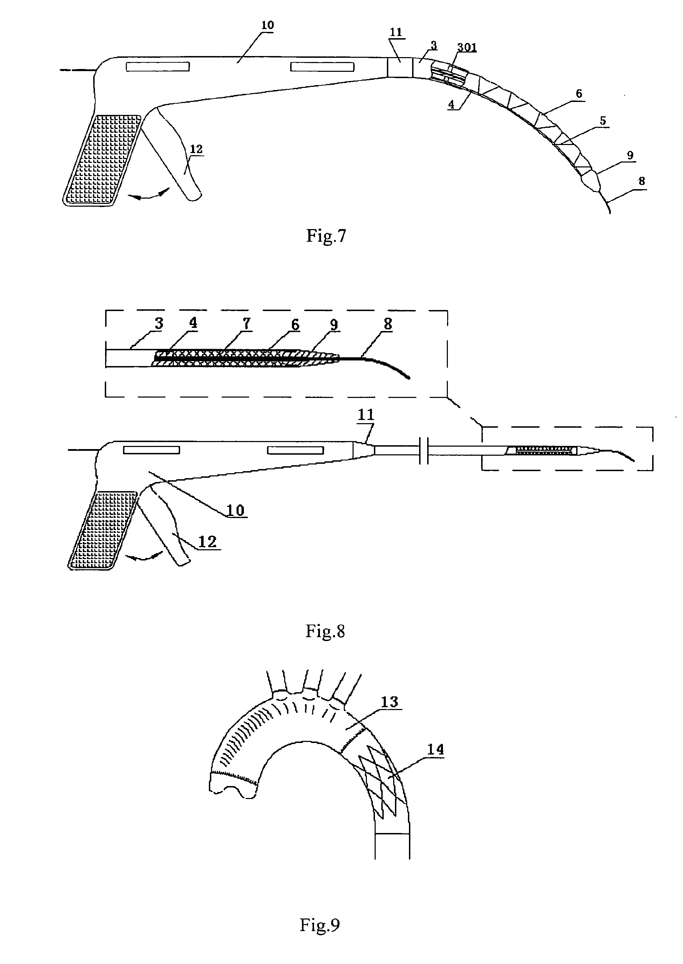

As shown in Fig.8, it is also a good way to design the delivery system as sheath form, i.e. sheath form delivery system. This system comprises a delivery sheath 3 and a push shaft 4. This delivery system comprises further a core bar 7 with a core bar hole, wherein said core bar is provided with a bullet-like tip 9 at its head, and the other end of the core bar 7 extends into the push shaft 4 and connected fixedly with the push shaft 4. Said push shaft 4 is provided with a through hole through which external guide wire 8 can extend over the tip 9. The above sheath form delivery system can be improved by adding a pistol-like body 10, connecting fixedly said delivery sheath 3 at the end with the muzzle 11 of the pistol-like body 10, and connecting fixedly the push shaft 4 with the trigger 12 of the pistol. The stent graft 6 is pre-compressed between the delivery sheath 3 and the core bar 7. In operation, insert the delivery sheath 3 into the cut port of the aortic vessel to be treated, pull the trigger 12 to make the push shaft 4 pressed against the stent graft 6, and withdraw the delivery sheath, thereby the stent graft 6 can be released gradually from the delivery sheath 3 and expand automatically. And then scissor the cut port to be aligned and overlapped with the end of the surgical stent graft. The surgery is finished after sewing a piece of synthetic vessel replacing the aorta and this overlapping port.

### Example 1

As shown in Fig. 9, the patient suffers from aorta aneurysm pathology, including ascendens aortae, arcus aorta and descending thoracic aorta. The central breast open surgery is performed: substitute synthetic vascular graft 13 with three branches for the ascendens aorta and arcus aorta; sew one end of the synthetic vascular graft together with ascendens aorta of the patient; insert a surgical stent graft 14 into the descending thoracic aorta and make it release and expand; sew the other end of the synthetic vascular graft together with the overlapping port of the descending thoracic aorta on the stent graft; and then sew the three branches of the synthetic vascular graft together with the corresponding blood vessels (i.e. anonyma artery, communis carotis artery, subclavian artery). Then the surgery is finished wholly.

### Example 2

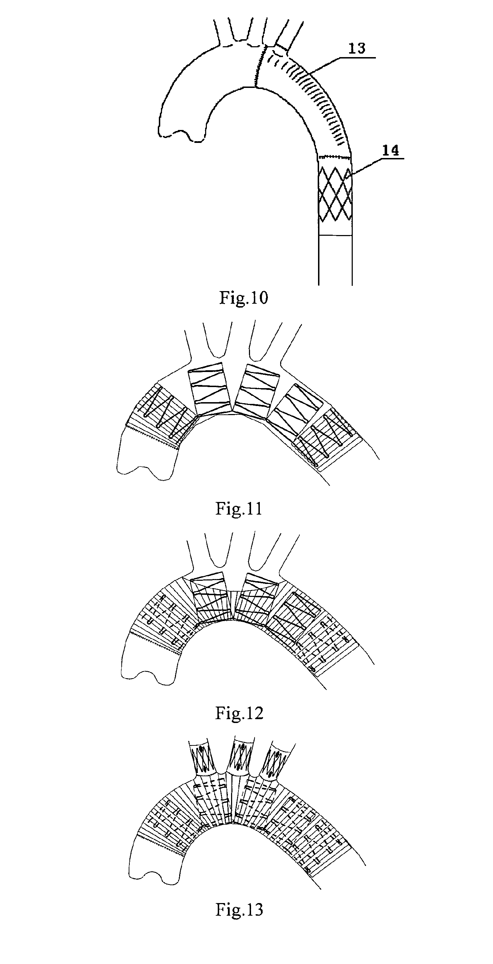

As shown in Fig.11 and Fig.12, the patient suffers from aorta aneurysm pathology, including ascendens aortae, arcus aorta and descending thoracic aorta. The central breast open surgery is performed: cut a port crossing the ascendens aorta; insert a surgical stent graft 14 as shown in Fig.2 or Fig.3 into the descending thoracic aorta; make the middle bare part of the stent graft face the three branches of the arcus aorta; sew the overlapping port of the stent graft and the ascendens aorta together with the cut port of the ascendens aorta towards the patient's heart. Then the surgery is finished wholly.

### Example 3.

As shown in Fig.10, the patient suffers from aorta aneurysm pathology, including descending thoracic aortic, abdominal aorta and left subclavian. The opening port in the surgery is under the fourth rib of the patient. The pathological vessel in the descending thoracic aorta was replaced by synthetic vessel with one branch and sew it respectively with corresponding arcus aorta cutting port and left subclavian port; insert a stent graft as shown in Fig. 1 into abdominal aorta port and release it as shown in Fig. 10; overlap the stent graft and the abdominal aorta port and sew the stent graft together with the other end of the synthetic vessel. Then the surgery is finished wholly.

### Example 4

As shown in Fig. 13, the patient suffers from aorta aneurysm pathology, including ascendens aortae, arcus aorta and descending thoracic aorta. The central breast open surgery is performed: cut a port crossing the ascendens aorta; insert a surgical stent graft 14 as shown in Fig.2, Fig.3 or Fig.4 into the descending thoracic aorta and make three branches in the middle of the surgical stent thrusted into the corresponding three branches of arcus aorta; sew the overlapping port of the stent graft and the ascendens aorta together with the cut port of the ascendens aorta towards the patient's heart. Then the surgery is finished wholly.

## Claims

1. A surgical stent graft comprising a synthetic vascular graft and a self-expendable stent which is fixed inside the synthetic vascular graft, wherein the stent's outer wall keeps in contact with the inner wall of the synthetic vascular graft and the vascular graft's one end is longer than the corresponding end of the self-expandable stent.

2. The surgical stent graft of claim 1 wherein both two ends of the synthetic vascular graft are longer than the corresponding ends of the stent.

3. The surgical stent graft of claim 1 wherein there is a window in the vascular graft, which divides the synthetic vascular graft and the stent respectively into two parts, and the two parts of the self-expandable stent are connected by alloy wire, the curvature of the wire is the same as that of the vascular graft and the wire can be seen through the window.

4. The surgical stent graft of claim 1 wherein there is a window in the vascular graft in the overlapping area of the stent-graft and the self-expandable stent. The self-expendable stent is visible through the window.

5. The surgical stent graft of claim 4 wherein the window is arranged cylindrically along the circumference of the synthetic vascular graft and divides the synthetic vessel into two separate parts, and the self-expendable stent is visible through the window.

6. The surgical stent graft of claim 1 wherein the synthetic vascular graft has 1-3 branch vessels.

7. The surgical stent graft of one of claims 1 to 6 wherein the synthetic vascular graft is made of the material chosen from PET or PTFE, and the stent is made of elastic metal materials chosen from NiNi shape memory alloy, CoCr alloy and 316L/317L stainless steel.

8. A delivery system used to deliver the surgical stent graft as claimed in one of claims 1 to 7, comprising a handle with one end having a tube port, a shaft and a piece of string with one end fixed on the handle and the other end fixed on the shaft, wherein one end of the stent graft is inserted into the tube port and bound on the shaft by the string, and said shaft extends through the tube port, and wherein the delivery system further comprises a core bar with a core bar hole through which external guide wire can extend from the tip, wherein said core bar is provided with a bullet-like tip at its head, and said handle is provided with a through hole in which the end of said core bar is fixed.

9. The delivery system of claim 8 wherein it comprises further a pistol-like body, said handle is connected fixedly at the end with the muzzle, and the end of the shaft is connected fixedly with the trigger of the pistol.

10. A delivery system used to deliver the surgical stent graft as claimed in one of claims 1 to 7, comprising a delivery sheath and a push shaft, wherein the delivery system comprises further a core bar with a core bar hole, wherein said core bar is provided with a bullet-like tip at its head, the other end of the core bar extends into the push shaft and connected fixedly with the push shaft, and the push shaft is provided inside with a through hole through which external guide wire can extend from the tip.

11. The delivery system of claim 10 wherein it comprises further a pistol-like body, said delivery sheath is connected fixedly at the end with the muzzle of the body, and the push shaft is connected fixedly with the trigger of the pistol.
